(19) Europäisches Patentamt
European Patent Office
Office européen des brevets

(11) EP 3 902 511 B1

(12) EUROPEAN PATENT SPECIFICATION

(45) Date of publication and mention of the grant of the patent:
**24.04.2024 Bulletin 2024/17**

(21) Application number: **19805986.7**

(22) Date of filing: **25.11.2019**

(51) International Patent Classification (IPC):
***A61K 8/24*** *(2006.01)* ***A61Q 11/00*** *(2006.01)*
***A61K 8/90*** *(2006.01)*

(52) Cooperative Patent Classification (CPC):
**A61Q 11/00; A61K 8/24; A61K 8/90**

(86) International application number:
**PCT/EP2019/082360**

(87) International publication number:
**WO 2020/135952 (02.07.2020 Gazette 2020/27)**

# (54) AN ORAL CARE COMPOSITION

MUNDPFLEGEZUSAMMENSETZUNG

COMPOSITION DE SOIN ORAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2018 EP 18215831**

(43) Date of publication of application:
**03.11.2021 Bulletin 2021/44**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **UNILEVER GLOBAL IP LIMITED**
  **Wirral**
  **Merseyside CH62 4ZD (GB)**
  Designated Contracting States:
  **CY DE GB IE IT MT RS TR**

(72) Inventors:
- **ALLAHBASH, Shahin**
  **Bangalore 560 066 (IN)**
- **BARNE, Sameer, Keshav**
  **Bangalore 560 066 (IN)**
- **VAIDYA, Ashish, Anant**
  **Bangalore 560 066 (IN)**

(74) Representative: **Tansley, Sally Elizabeth**
**Unilever Patent Group**
**Bronland 14**
**6708 WH Wageningen (NL)**

(56) References cited:
**US-A- 4 370 314**
**US-A1- 2003 191 209**
**US-A1- 2006 171 907**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the Invention

The present invention relates to an oral care composition for minimizing staining of teeth.

### Background of the Invention

Long-lasting whitening of teeth is of considerable interest to consumers. Foods and drinks such as tea, coffee and wine may form dental stains by directly depositing chromogens on the tooth surface. Attraction of materials to the tooth surface plays a critical role in the deposition of extrinsic dental stain. The chromogens in these beverages that are responsible for causing dental stain are known as tannins and are composed of polyphenols such as catechins. These materials generate color due to the presence of conjugated double bonds and are thought to interact with the tooth surface via an ion exchange mechanism.

Traditional tooth whitening methods involve either peroxide bleaching from kit formats or abrasive stain removal from toothpaste formats. These particles are essentially insoluble particles that remove extrinsic stain from the surface of the tooth via abrasion when applied with a brush. The present invention relates to a novel oral care composition that delivers effective levels of stain protection and prolongs the effects of tooth whitening by preventing staining of teeth. The invention discloses a stain repellent coating having a synergistic blend of a hydrophilic-hydrophobic block co-polymer and $Ca^{2+}$ chelating agent (a phosphate salt) at a defined ratio. This helps to provide a protective stain coating on the tooth surface during the consumption of food/drinks post whitening treatments.

The block copolymers used in the present invention are what are generally known as Pluronics. These polymers, in general, have been known to be used in oral care. The phosphate salt used in the present invention is hexametaphosphate which has also been used in oral care.

US2014377194A (P&G) discloses an oral care composition containing (a) a zinc citrate; and (b) a surface-active organophosphate compound and it also relates to a method of preventing a stain from depositing on a tooth surface or other oral surfaces.

US2003124065A (P&G) discloses an oral care composition and methods for overall cleaning, whitening and preventing, reducing or removing surface deposited stains on natural teeth and dental prosthesis, the compositions comprising in an orally acceptable carrier at least 0.1% by weight of a water-soluble or water-dispersible copolymer prepared by copolymerizing one or a mixture of vinyl pyrrolidone (VP) monomers with one or a mixture of C1-C19 alkyl carboxylic acid (AC) C2-C12 alkenyl ester monomers; preferably, these compositions further comprise one or a mixture of other oral care agents selected from a water soluble alkali metal or ammonium tripolyphosphate in an amount at least about 0.5% by weight of the composition, an abrasive, preferably a precipitated silica abrasive, in an amount at least about 6% by weight of the composition and a bleaching agent in an amount at least about 0.1% by weight of the composition.

Although these two individual ingredients have, in general, been used in oral care compositions, the present invention derives synergistic benefit in selection of specific pluronics where the ethoxylate part of the polymers is in a selective amount (from 10 to 90%) of the total polymer. Further it is specifically required that the weight ratio of the polymer to the hexametaphosphate is from in a specific range (1:4 to 4:1) and does not work well outside this range.

Furthermore, the total amount of polymer and hexametaphosphate is at most 2 wt%. It is these specific polymer ratios, weight ratios and total amount of the ingredients which lends the synergistic activity of this combination to the present invention.

US4370314 A1 (Colgate, 1975) discloses oral care compositions comprising hexametaphosphate as stain reducing additive. Surfactants are also used. Examples show mouthwash compositions comprising Pluronic® and sodium hexametaphosphate,

US2006171907 A1 (P&G) discloses an oral care composition having enhanced whitening and stain prevention with polyphosphate and tooth bleaching agent. This publication teaches buffering of the composition at pH 4.0 to 6.0 for peroxide stability.

US2003191209 A1 (Guan Yue Hugh) discloses the use of a combination of poloxamers for the prevention of adherence of plaque and/or stains on the teeth. The subject matter of the claims is thus novel.

It is thus an object of the present invention to provide for an oral care composition that minimizes staining of teeth.

### Summary of the Invention

According to the first aspect of the present invention there is provided an oral care composition comprising:

(a) 0.1 to 2%, by weight of the composition, a polymer having the structure

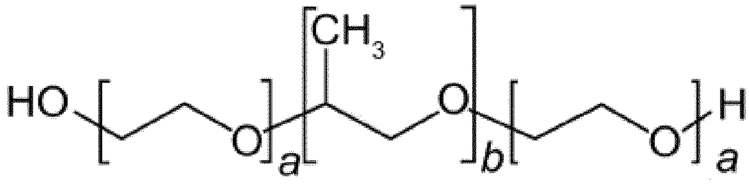

where the molecular weight ratio of ethoxylate groups to the propoxylate groups is in the range of 0.1 to 3.0; the molecular weight of the polymer is from 1700 to 15000 Da;
(b) hexametaphosphate; and
(c) an orally acceptable base;

wherein the weight ratio of the polymer to hexametaphosphate is from 1:4 to 4:1; and wherein the pH of the composition is between 6.1 and 9.0;
wherein the total amount of polymer and hexametaphosphate is at the most 2% by weight of the composition.

## Detailed Description of the Invention

Except in the examples, or where otherwise explicitly indicated, all numbers in this description indicating amounts of material or conditions of reaction, physical properties of materials and/or use may optionally be understood as modified by the word "about". All amounts are by weight of the final composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

"Oral care composition" for the purposes of the present invention means a paste, powder, liquid, gum, serum or other preparation for cleaning the teeth or other surfaces in the oral cavity.

"Tooth paste" for the purpose of the present invention means a paste or gel dentifrice for use with a toothbrush. Especially preferred are tooth pastes suitable for cleaning teeth by brushing for about one minutes, preferably two minutes.

"Mouth wash" for the purpose of the present invention means liquid dentifrice for use in rinsing the mouth. Especially preferred are mouth washes suitable for rinsing the mouth by swishing and/or gargling for about half a minute before expectorating.

The present invention relates to an oral care composition comprising a polymer having the structure of compound of formula 1

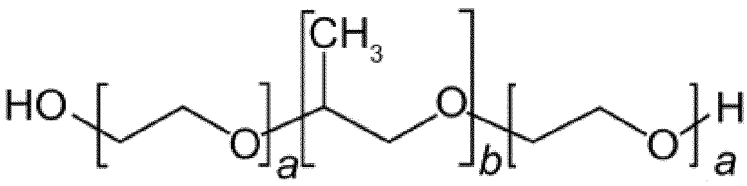

Compound of formula 1.

In the above compound, the molecular weight ratio of ethoxylate groups to the propoxylate groups is in the range of 0.1 to 3.0, preferably in the range of 0.1 to 2.5. The molecular weight of the polymer is in the range of from 1700 to 15000 Da, preferably in the range of 10000 to 13000 Da. The present invention preferably requires polymers, which contains both EO and PO (propoxylate) parts, with the above structure. The ethoxylate (EO) part of the polymer is to preferably be from 10 to 80% of the total polymer, more preferably from 10 to 60% of the total polymer. Commercially

available polymers which are preferably used in the composition of the present invention include Pluronic L-61, Pluronic F88, Pluronic F77, Pluronic F108, or Pluronic 127, more preferably Pluronic 127. The polymer is present in 0.1 to 2%, preferably 0.5 to 1.5% by weight of the composition. The above range is especially applicable when formulated as a toothpaste. When formulated as a mouthwash, the polymer is preferably included in lower amount, e.g. in the range of 0.1 to 0.7%, more preferably 0.1 to 0.5% by weight of the composition.

The composition of the invention includes a phosphate compound which is hexametaphosphate. Sodium hexametaphosphate has the structure as give below:

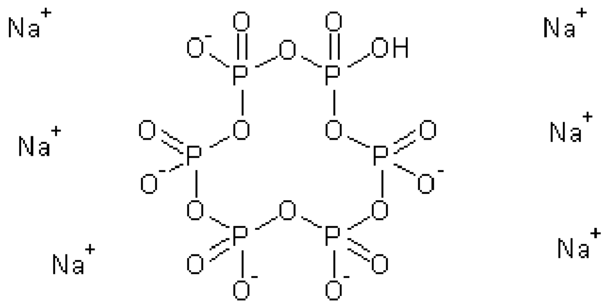

The hexametaphosphate is preferably included in 0.1 to 2%, more preferably 0.1 to 1.0% by weight of the composition.

It is required as per the present invention that the weight ratio of polymer to hexametaphosphate is from 1:4 to 4:1. In a preferred aspect this ratio is from1:3.5 to 3.5:1, further preferred ratio being from 1:3 to 3:1.

The total amount of polymer and hexametaphosphate in the composition of the invention is at the most 2% by weight of the composition. The above range is especially applicable when formulated as a toothpaste. The present inventors have observed that at higher than this amount, the anti-staining efficacy reduces. When formulated as a mouthwash, the total amount of polymer and hexametaphosphate is preferably included in lower amount, e.g. in the range of 0.1 to 0.75%, more preferably 0.2 to 0.75% by weight of the composition.

Without wishing to be bound by theory, the present inventors believe that it is the synergistic interaction of the two ingredients of which the polymer modifies the hydroxyapatite surface of the tooth to provide a low interfacial energy to it, so as to repel stains from the surface and the calcium binding affinity of the phosphate to the tooth surface which at the specific weight ratios of the ingredients and the specifically chosen polymer structure within the chosen EO/PO ratio that provides the desired benefit of the present invention.

The present inventors have found that the present invention is capable of minimizing staining of teeth without the use of conventional whitening agents like bleaches. Thus, the composition of the present invention preferably comprises less than 0.5, more preferably less than 0.2%, furthermore preferably less than 0.1%, and ideally less than 0.1% bleach by weight of the composition. Optimally, the present invention does not include any bleach. By bleach is meant a compound that produces a bleaching or whitening action on a substrate like teeth usually by way of oxidation. Example of common bleaches used in oral care include peroxides like hydrogen or calcium peroxide and and percarbonates like sodium percarbonate.

The composition of the invention comprises an orally acceptable base. The orally acceptable base preferably comprises an abrasive, a humectant, a surfactant, water, volatile alcohol or combinations thereof. The orally acceptable base preferably makes up 96 to 99.8% by weight of the composition.

The oral care composition of the present invention may be delivered in the form of an ointment, a gel, a dentifrice or a mouthwash. Dentifrices include forms like toothpaste and toothpowder. A composition is most preferably presented in the form of a toothpaste, a toothpowder or a mouthwash; most preferably in the form of a toothpaste or a mouthwash.

Oral care composition of the present invention in the form of a toothpaste preferably comprises an abrasive. Gels usually contain silica, whereas opaque creams generally contain calcium based abrasives, especially chalk. It is preferred that the composition of the invention comprises silica as the abrasive. Such a composition comprising abrasive silica is generally in the form of a gel. Abrasive silica for incorporation in the composition of the invention is preferably one with a low refractive index. It may be used as the sole abrasive silica, or in conjunction with a low level of other abrasive silicas, e. g. those according to EP236070. The low refractive index silicas , used as abrasives in the present invention are preferably silicas with an apparent refractive index (RI) in the range of 1.41 to 1.47, preferably 1.435 to 1.445 , preferably having a weight mean particle size of between 5 and 15 mm, a BET (nitrogen) surface area of between 10 and 100 $m^2/g$ and an oil absorption of about 70 - 150 $cm^3/100$ g, but abrasive silicas with a lower apparent refractive index may also be used . Typical examples of suitable low refractive index abrasive silicas (e.g. having an RI of between 1.435 and 1.445) are Tixosil 63 and 73 ex Rhone Poulenc ; Sident 10 ex Degussa; Zeodent 113 ex Zeofinn; Zeodent 124 ex Huber, Sorbosil AC 77 ex Crosfield Chemicals (having an RI of approximately 1.440). The amount of these silicas

in the composition of the present invention generally ranges from 5-60% by weight, usually 5-20%, most preferably from 10 to 17% by weight of the composition. When the toothpaste is formulated as an opaque cream, chalk or calcium carbonate may be included as an abrasive in from 15% to 70%, more preferably from 30% to 70% most preferably from 30 to 60 wt% by total weight of the composition.

In a preferred embodiment, the composition comprises a thickener. Thickeners that may be used in this invention include, sodium carboxymethyl cellulose (SCMC), hydroxyl ethyl cellulose, methyl cellulose, ethyl cellulose, gum tragacanth, gum arabic, gum karaya, xanthan gum, sodium alginate, carrageenan gum, guar gum, Irish moss, starch, modified starch, silica based thickeners including silica aerogels, magnesium aluminum silicate (e.g., Veegum), Carbomers (cross-linked acrylates) and mixtures thereof.

Typically, thickening silica, sodium carboxymethyl cellulose and/or a Carbomer is/are preferred thickeners for use in the composition of the invention. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickening silica is especially preferred to be used in gel toothpastes.

Gel toothpastes generally contain upto 8.5 wt% thickening silica whereas opaque toothpastes typically contain 3 to 4 wt% thickening silica.

When present, preferred thickening silicas include AEROSIL T series from Degussa or the CAB-O-SIL series from Cabot Corporation, silica gels such as the SYLODENT or SYLOX series from W. R.Grace & Co or precipitated silica such as ZEOTHIX 265 from J. M. Huber Corporation. Useful silica thickeners also include ZEODENT 165, ZEODENT 163 and/or 167 and ZEOFREE 153, 177, and/or 265 silicas, all available from J. M. Huber Corporation. Other preferred thickening silicas include MFIL, MFIL-P (From Madhu Silica, India), SIDENT 22 S and AEROSIL 200 (Ex. Evonik Industries), SYLODENTand PERKASIL thickening silicas from WR Grace & Company and Tixosil 43 and 331 from Rhodia, synthetic finely divided pyrogenic silica such as those sold under the trademarks SYLOID 244, SYLOID 266 and AEROSIL D- 200.

Thickener, when present, preferably makes up from 0.01 to about 10%, more preferably from 0.1 to 9%, and most preferably, from 1.5 to 8% by weight of the composition.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof, are the preferred humectants, most preferred ones being glycerol (also known as glycerine) and sorbitol.

The humectant may be present in the range of from 10 to 90% by weight of oral care compositions. More preferably, the humectant makes up from 25 to 80%, and most preferably, from 45 to 70% by weight of the composition, based on total weight of the composition and including all ranges subsumed therein.

Preferably, an oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. More preferably the surfactant comprises or is an anionic surfactant. The preferred anionic surfactants are sodium lauryl sulphate and/or sodium dodecylbenzene sulfonate. Most preferably the surfactant is sodium lauryl sulphate. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Most preferred surfactants are an alkali metal alkyl sulphate or a betaine.

Water may preferably be included in 5 to 95%, in particular 10 to 75%, and especially at from 10 to 60%, furthermore preferably 10 to 50%, most preferably 40 to 50% by total weight of the composition.

When the oral care composition of this invention is a toothpaste or gel, the same typically has a viscosity from about 30,000 to 180,000 centipoise, and preferably, from 60,000 to 170,000 centipoise, and most preferably, from 65,000

to 165,000 centipoise, as measured at various finite shear rates To quantity the zero shear viscosity, an Anton Paar rheometer with parallel plate geometry is used. Steady state flow experiments are carried out by varying the shear rate from 0.1 $s^{-1}$ to 100 $s^{-1}$. From the calibration chart so produced the zero shear viscosity of the composition is measured. The zero shear viscosity is found to be in the range of 500 to 1500 Pa-s. Similarly, for a mouthwash composition the zero shear viscosity is measured to be in the range of 100 to 200 mPa-s.

The pH of the composition is preferably between 6.0 and 9.0, more preferably in the range of 6.1 to 8.5, furthermore preferably in the range of 6.1 to 8.0, most preferably in the range of 6.2 to 7.5.

The pH was measured using an Orion Star A 215 (Thermo scientific) advanced bench top pH meter. The pH meter was calibrated with a 3-point calibration at pH 2, 7 and 10. The relative accuracy of the pH meter is 0.002 units. All measurements were carried out at 25 °C.

The toothpaste samples were diluted with DI water at a ratio of 1: 3 (1 part of toothpaste: 3 parts water) prior to pH measurements. The pH of the mouth spray was measured with no dilution.

The oral care composition of the present invention may contain a variety of other ingredients which are common in the art to enhance physical properties and performance. These ingredients include antimicrobial, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The invention will now be illustrated with the help of the following non-limiting example.

## Examples

Examples 1-8: Effect of combination of the polymer as per the invention and hexametaphosphate in ratios within the invention and outside the invention

The pellicle coated HAP discs were treated with the dentifrice (with base composition as given in Table - 1 below) containing various stain repellent ingredients as listed in Table - 2. The procedure used to simulate staining of teeth and the whiteness of the disks was measured as detailed below:
HAP disks were soaked in 2 ml saliva/water and placed in the oven at 37°C for 30 minutes. The disks were rinsed with milli Q water and dabbed to remove excess water Pellicle coating HAP disks (moist) coated were soaked with 2 ml of tooth paste slurry or model base for 5 minutes. Disks were brushed for 1 minute and rinsed with 100 ml Milli Q water. Initial L*, a*, b* values were monitored using a spectrophotometer. Treated HAP disks were soaked in 2 % tea solution (2 grams black tea in 98 ml boiling water and stirred for 5 minutes) at 37°C for 10 minutes. Staining cycle was repeated for 3 times and the stained HAP disks were brushed/ rinsed after each staining cycle. The L*, a*, b* values were monitored after each staining cycle. Delta E value was then calculated using the following equation.

$$\Delta E = \sqrt{((L * \text{initial} - L * \text{final})^2 + (a * \text{initial} - a * \text{final})^2 + (b * \text{initial} - b * \text{final})^2)}$$

**Table 1**

| Ingredients | |
|---|---|
| Sorbitol 70% | 45.0 |
| Sodium fluoride | 0.32 |
| Saccharine | 0.20 |
| SCMC | 0.52 |
| Abrasive silica | 1.65 |
| Absil 100 | 8,0 |
| SLS | 1.7 |
| Polymer - Pluronic F127 | See Table - 2 |

(continued)

| Ingredients | |
|---|---|
| Hexametaphosphate (HMP) | See Table - 2 |
| Water | To 100 |

The ∆E values of HAP disks treated with the various compositions is given in the Table 2 below:

Table 2

| Examples | Dentifrice Composition comprising | ∆E |
|---|---|---|
| 1 | No polymer and no HMP | 40.8 |
| 2 | 2% HMP | 28.7 |
| 3 | 5% HMP | 24.4 |
| 4 | 2% Polymer | 30.9 |
| 5 | 1.75% Polymer + 0.25% HMP | 32.2 |
| 6 | 0.25% Polymer + 1.75% HMP | 23.0 |
| 7 | 1.5% Polymer + 0.5% HMP | 15.9 |
| 8 | 0.5% Polymer + 1.5% HMP | 14.9 |

In the table above, Polymer refers to Pluronic-127 sourced from Sigma and HMP refers to sodium hexametaphosphate sourced from Sigma.

The data in the Table 1 above indicates that oral care compositions as per the invention (Example 7 and 8) provides vastly superior anti-staining efficacy (as evident by the low ∆E values) as compared to compositions outside the invention. (Examples 1 to 6).

Examples 9-10: Effect of including total amount of polymer and HMP higher than 2 wt%:

Pellicle coating HAP disks (moist) coated were soaked in 2 ml model base (45 % Sorbitol and 1.8 wt% SDS) additionally containing polymer and HMP as given in Table 3 for 5 minutes. Disks were brushed for 1 minute and rinsed with 100 ml Milli Q water. Initial L*, a*, b* values were monitored using a spectrophotometer. Treated HAP disks were soaked in 2 % tea solution (2 grams black tea in 98 ml boiling water and stirred for 5 minutes) at 37°C for 10 minutes. Staining cycle was repeated for 3 times and the stained HAP disks were brushed/ rinsed after each staining cycle. The L*, a*, b* values were monitored after each staining cycle. ∆E value was then calculated.

Table 3

| Example | Composition | ∆E |
|---|---|---|
| 9 | 2 wt % Polymer + 1wt % HMP | 35.2 |
| 10 | 2 wt % Polymer + 2 wt % HMP | 39.2 |

The data in the Table 3 above indicates that having a combination of polymer and HMP higher than 2 wt% in a model composition does not give good benefit.

Examples 11-14: Effect of including phosphates other than HMP:

Experiments were conducted using phosphates other than HMP. The procedure used was similar to that used in generating data for Table - 3 above. The ∆E value for the various examples are summarized in table -4 below:

**Table 4**

| Example | Composition | ΔE |
|---|---|---|
| 11 | 45% Sorbitol+1.8 %SDS | 27.9 |
| 12 | Control + 2 wt % disodium hydrogen phosphate | 26.4 |
| 13 | Control + 2 wt % Trisodium phosphate | 34.4 |
| 14 | Control + 2 wt % HMP | 22.4 |

The data in the Table 4 above indicates that HMP (example 14) provides superior anti-staining benefit as compared to Examples (11 to 13) where other phosphates were used.

## Claims

1. An oral care composition comprising

(a) 0.1 to 2% by weight of the composition, a polymer having the structure

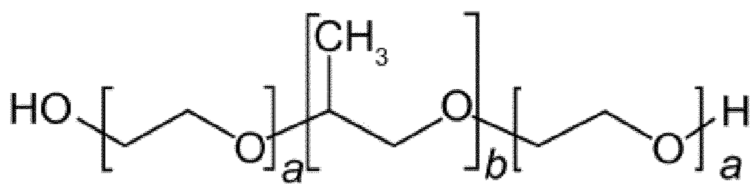

where the molecular weight ratio of ethoxylate groups to the propoxylate groups is in the range of 0.1 to 3.0; the molecular weight of the polymer is from 1700 to 15000 Da;
(b) hexametaphosphate; and
(c) an orally acceptable base;
wherein the weight ratio of the polymer to hexametaphosphate is from 1:4 to 4:1; and wherein the pH of the composition is between 6.1 and 9.0;
wherein the total amount of polymer and hexametaphosphate is at the most 2% by weight of the composition.

2. A composition as claimed in claim 1 wherein the weight ratio of polymer to hexametaphosphate is from 1:3 to 3:1.

3. A composition as claimed in any one of the preceding claims wherein the molecular weight of the polymer is from 10,000 to 13,000 Da.

4. A composition as claimed in any one of the preceding claims comprising 0.1 to 2% hexametaphosphate.

5. A composition as claimed in any one of the preceding claims wherein the orally acceptable base comprises an abrasive, a humectant, surfactant, water, volatile alcohol or combinations thereof.

6. A composition as claimed in any one of the preceding claims comprising 96 to 99.8% orally acceptable base.

7. A composition as claimed in claim 5 or 6 wherein the abrasive is silica.

8. A composition as claimed in any one of claims 5 to 7 wherein the humectant is a polyhydric alcohol preferably sorbitol or glycerol.

9. A composition as claimed in any one of the preceding claims wherein the composition is a toothpaste, a toothpowder or a mouthwash.

**Patentansprüche**

1. Mundpflegezusammensetzung, umfassend

(a) 0,1 bis 2 Gewichts-% der Zusammensetzung eines Polymers der Struktur

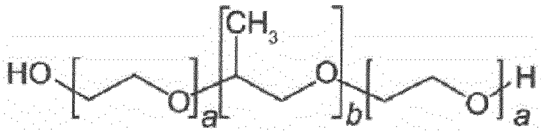,

wobei das Molekulargewichtsverhältnis der Ethoxylatgruppen zu den Propoxylatgruppen in dem Bereich von 0,1 bis 3,0 liegt, das Molekulargewicht des Polymers 1700 bis 15000 Da beträgt;
(b) Hexametaphosphat; und
(c) eine oral akzeptable Basis;
wobei das Gewichtsverhältnis von Polymer zu Hexametaphosphat 1:4 bis 4:1 beträgt und wobei der pH-Wert der Zusammensetzung zwischen 6,1 und 9,0 liegt;
wobei die Gesamtmenge von Polymer und Hexametaphosphat höchstens 2 Gewichts-% der Zusammensetzung beträgt.

2. Zusammensetzung, wie im Anspruch 1 beansprucht, wobei das Gewichtsverhältnis von Polymer zu Hexametaphosphat 1:3 bis 3:1 beträgt.

3. Zusammensetzung, wie in irgendeinem der vorhergehenden Ansprüche beansprucht, wobei das Molekulargewicht des Polymers 10.000 bis 13.000 Da beträgt.

4. Zusammensetzung, wie in irgendeinem der vorhergehenden Ansprüche beansprucht, umfassend 0,1 bis 2% Hexametaphosphat.

5. Zusammensetzung, wie in irgendeinem der vorhergehenden Ansprüche beansprucht, wobei die oral akzeptable Basis ein Schleifmittel, ein Feuchthaltemittel, Tensid, Wasser, flüchtigen Alkohol oder Kombinationen davon umfasst.

6. Zusammensetzung, wie in irgendeinem der vorhergehenden Ansprüche beansprucht, umfassend 96 bis 99,8% oral akzeptable Basis.

7. Zusammensetzung, wie im Anspruch 5 oder 6 beansprucht, wobei das Schleifmittel Siliciumdioxid ist.

8. Zusammensetzung, wie in irgendeinem der Ansprüche 5 bis 7 beansprucht, wobei das Feuchthaltemittel ein mehrwertiger Alkohol, vorzugsweise Sorbitol oder Glycerin, ist.

9. Zusammensetzung, wie in irgendeinem der vorhergehenden Ansprüche beansprucht, wobei die Zusammensetzung eine Zahnpasta, ein Zahnpulver oder ein Mundwasser ist.

**Revendications**

1. Composition pour le soin oral comprenant

(a) de 0,1 à 2 % en masse de la composition, d’un polymère ayant la structure

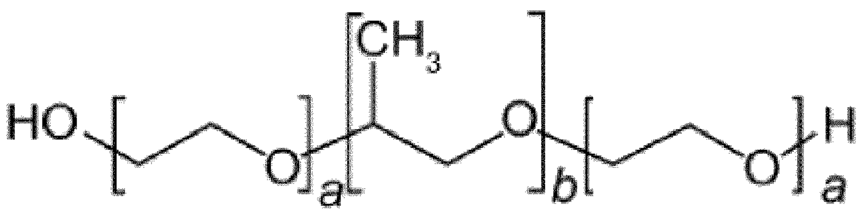

où le rapport de masse moléculaire des groupes éthoxylate aux groupes propoxylate se trouve dans l'intervalle de 0,1 à 3,0 ; la masse moléculaire du polymère est de 1 700 à 15 000 Da ;
(b) de l'hexamétaphosphate ; et
(c) une base oralement acceptable ;
dans laquelle le rapport en masse du polymère à l'hexamétaphosphate est de 1:4 à 4:1 ; et
dans laquelle le pH de la composition est de 6,1 à 9,0 ;
dans laquelle la quantité totale de polymère et d'hexamétaphosphate est d'au plus 2 % en masse de la composition.

2. Composition selon la revendication 1, dans laquelle le rapport en masse de polymère à hexamétaphosphate est de 1:3 à 3:1.

3. Composition selon l'une quelconque des revendications précédentes, dans laquelle la masse moléculaire du polymère est de 10 000 à 13 000 Da.

4. Composition selon l'une quelconque des revendications précédentes comprenant de 0,1 à 2 % d'hexamétaphosphate.

5. Composition selon l'une quelconque des revendications précédentes, dans laquelle la base oralement acceptable comprend un abrasif, un humectant, un tensioactif, de l'eau, un alcool volatil ou des combinaisons de ceux-ci.

6. Composition selon l'une quelconque des revendications précédentes comprenant de 96 à 99,8 % de base oralement acceptable.

7. Composition selon la revendication 5 ou 6, dans laquelle l'abrasif est la silice.

8. Composition selon l'une quelconque des revendications 5 à 7, dans laquelle l'humectant est un alcool polyhydrique, de préférence le sorbitol ou glycérol.

9. Composition selon l'une quelconque des revendications précédentes, dans laquelle la composition est un dentifrice, une poudre dentaire ou une eau de douche.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014377194 A
- US 2003124065 A
- US 4370314 A1
- US 2006171907 A1
- US 2003191209 A1
- EP 236070 A


### Non-patent literature cited in the description


- *CHEMICAL ABSTRACTS,* 9063-87-0
- *CHEMICAL ABSTRACTS,* 9004-32-4